# EUROPEAN PATENT SPECIFICATION

(11) **EP 3 881 801 B1**
(45) Date of publication and mention of the grant of the patent: **23.04.2025**
(21) Application number: 20163990.3
(22) Date of filing: 18.03.2020
(51) Int. Cl.: A61F 2/24, A61F 2/95

(54) **IMPROVEMENTS IN OR RELATING TO THE DELIVERY AND UNSHEATHING OF PROSTHETIC HEART VALVES**
VERBESSERUNGEN AN ODER IM ZUSAMMENHANG MIT DER ABGABE UND ENTMANTELUNG VON HERZKLAPPENPROTHESEN
AMÉLIORATIONS APPORTÉES OU SE RAPPORTANT À LA POSE ET LE DÉGAINAGE DE VALVULES CARDIAQUES PROTHÉTIQUES

(43) Date of publication of application: 22.09.2021
(73) Proprietor: Medtronic Inc., Minneapolis, MN 55432 (US)
(72) Inventor: ALLELEYN, Luc Johannes Martin, 6286 CG Wittem (NL)
(74) Representative: Gray, James

(56) References cited:
- WO-A1-2017/117109
- WO-A2-2013/028387
- US-A1- 2014 371 844

## Description

The present disclosure relates to the delivery and unsheathing of prosthetic heart valves at desired locations in a human heart.

It is a known to treat problems that might occur in one or more valves of a human heart, such as stenosis, by replacement of the affected valve or valves with an appropriately configured prosthetic heart valve. Replacement of the mitral, aortic, tricuspid and pulmonary valves is possible. In each case, replacement of an affected valve generally involves the delivery of a prosthetic valve in a volume reduced or collapsed state to a desired position within the heart which is at or adjacent to the location of the affected valve. The prosthetic valve is then manipulated so as to adopt a volume increased or non-collapsed state so as to be superimposed over, and take the function of, the affected valve.

Taking the example of the mitral valve, a known prosthetic mitral valve is arranged to regulate blood flow between two chambers, the left atrium and left ventricle of a human heart. The prosthetic mitral valve comprises a stent, a cylindrical cuff, leaflets and a flange formed of braided nitinol wires. The leaflets are operable between a closed status in which the leaflets prevent the flow of blood from the left ventricle to the left atrium and an open status in which the leaflets maximally enable the flow of blood through the prosthetic valve, i.e. the leaflets restrict the flow of blood through the prosthetic valve minimally. The prosthetic mitral valve is collapsible for easy delivery and expandable in radial direction to be anchored and functioning in the heart. The flange itself is formed of two overlaying sheets that are joined together. The flange is formed of a braided material.

The flange comprises a flared portion and a body portion and is connected to the stent at a coupling portion. On the side of the ventricle the stent protrudes into the ventricle beyond the coupling portion. On the atrial side of the prosthetic heart valve the flared portion is directly connected to the coupling portion, whereas at the ventricular side the body portion is between the coupling portion and the flared portion. This means that the prosthetic heart valve has an asymmetric configuration about a central longitudinal axis because the flange forms different shapes on the atrial and ventricular sides of the prosthetic heart valve. Consequently, different portions of the stent on the ventricular side are exposed. In addition, the prosthetic mitral valve has anchoring arms connected to the stent on the atrial side, whereas it has stabilizing wires connected to the flange on the ventricular side for anchoring.

Preferably a prosthetic mitral valve is delivered via a catheter in a procedure sometimes referred to as transcatheter mitral valve replacement (TMVR) wherein transcatheter refers to the delivery by catheter. Transcatheter mitral valve replacement is a relatively new field, and transseptal delivery of the TMVR device is becoming more preferred due to reduced invasiveness.

Typically, transseptal unsheathing of TMVR devices is conducted in a sub or intraannular fashion, meaning that part of the device is unsheathed inside the ventricle before the device is implanted on the target area. Insufficient left ventricular volume is a major exclusion criterion for TMVR devices -- the left ventricle should be able to accommodate the TMVR device during unsheathing without interfering with the chordal apparatus and/or ventricular endocardium. In addition, insufficient left ventricular volume can result increase the risk of obstruction of the left ventricular outflow tract (LVOT) by the TMVR device after deployment.

The above description of transcatheter mitral valve replacement is provided by way of example only and is not intended to be limiting upon the scope of the claimed invention. As noted above, other heart valves can be replaced. Taking the example of replacement of the aortic valve, transsubclavian or transfemoral delivery may be employed. With regard to replacement of the tricuspid valve, transjugular delivery may be employed. US2014/371844 discloses a prosthetic heart valve.

The present invention relates to the configuration of a prosthetic heart valve within a delivery device such as a catheter or a delivery capsule that enables supra-annular unsheathing of the prosthetic heart valve as opposed to sub-annular unsheathing.

According to a first aspect of the present invention there is provided a method of loading a prosthetic heart valve into a delivery device, the prosthetic heart valve including:
- an inner frame having a tubular shape; and
- a braided wire mesh arranged outside of the inner frame and defining a circumferential flange around the inner frame;
   the method comprising the steps of;
- providing a delivery device having a lumen defined by a wall and a distal aperture at a distal end thereof, the lumen having a diameter that is less than that of the inner frame and braided wire mesh;
- providing the heart valve in a radially uncompressed condition;
- positioning the prosthetic heart valve such that the inner frame is received through the distal aperture and into the lumen;
- moving the prosthetic heart valve fully into the lumen such that the inner frame and braided wire mesh are radially compressed; wherein the step of moving the braided wire mesh into the lumen of the delivery device includes inverting the circumferential flange.

The circumferential flange is inverted such that its orientation relative to the inner frame when the prosthetic heart valve is in the radially compressed condition is opposite to that when the prosthetic heart valve is in the radially uncompressed condition. More specifically, a circumferential surface of the flange which faces inwardly and towards the inner frame when the prosthetic heart valve is in the radially uncompressed condition is repositioned so as to face radially outwardly and away from the inner frame when the prosthetic heart valve is in the radially compressed condition. Similarly, a circumferential surface of the flange which faces outwardly and away from the inner frame when the prosthetic heart valve is in the radially uncompressed condition is repositioned so as to face radially inwardly and towards the inner frame when the prosthetic heart valve is in the radially compressed condition.

The loading of the prosthetic heart valve into the delivery device may utilise a tapered conduit having a first aperture, a second aperture and a tapered lumen extending between the first aperture and the second aperture, wherein the diameter of the first aperture is greater than the second aperture, and wherein further the diameter of the first aperture is greater than the diameter of the inner frame and less than the diameter of the circumferential flange.

In such an instance the method may comprise the further steps of:
- positioning the prosthetic heart valve relative to the first aperture of the tapered conduit such that inner frame is received through the first aperture and within the tapered lumen, and the circumferential flange surrounds the first aperture to the exterior of the tapered conduit;
- aligning the second aperture of the tapered conduit with the distal aperture of the delivery device;
- moving the prosthetic heart valve fully into the lumen such that the inner frame and braided wire mesh are radially compressed by the tapered lumen of the tapered conduit before the compressed valve passes into the lumen of the delivery device through the distal aperture of the delivery device

The step of inverting the circumferential flange includes the step of;
- moving the flange into engagement with the portion of the tapered conduit defining the first opening thereof.

It will be understood that the portion of the tapered conduit defining the first opening acts as a circumferential abutment surface which bears against the circumferential flange as the prosthetic heart valve is moved into the tapered lumen. The aforementioned circumferential abutment surface bears against the flange, and causes inversion of the flange, as the result of continued movement of the prosthetic heart valve into the tapered conduit.

The prosthetic heart valve may include a plurality of hooks which are attached to the inner frame and which overlie the braided wire mesh when the valve is in the radially uncompressed condition. In such an instance, the step of inverting the circumferential flange results in the braided wire mesh moving to a position where it overlies the hooks.

The step of moving the prosthetic heart valve into the lumen of the delivery device may be achieved by the use of traction wires which are releasably attachable to the inner frame of the valve. Alternatively, other methods may be employed to move the prosthetic heart valve into the lumen of the delivery device. These may include, but not be limited to, manual manipulation, the use of a plunger tool, or the use of an iris crimper.

The delivery device may be a catheter. Alternatively, the delivery device may be a capsule which is configured to retain the valve in radially compressed condition. The capsule may form an integral part of a catheter. Alternatively, the capsule may be mountable or otherwise connectable to a catheter.

According to another aspect of the present invention there is provided the combination of a delivery device and a prosthetic heart valve in a radially compressed condition and located within the delivery device, wherein the prosthetic heart valve includes:
- an inner frame having a tubular shape; and
- a braided wire mesh arranged outside of the inner frame and defining a circumferential flange around the inner frame, the circumferential flange having an outer circumferential periphery; and
   wherein the delivery device includes:
   - a lumen defined by a wall of the delivery device and a distal aperture; wherein
   - the inner frame and braided wire mesh are maintained in the radially compressed condition by the wall;
   - the circumferential flange is held in an inverted state; and
   - the part of the radially compressed valve that is most proximal to the distal aperture of the delivery device is the portion of the braided wire mesh that defines the outer circumferential periphery of the flange.

The part of the radially compressed valve that is most distal to the distal aperture of the delivery device may be the inner frame of the valve.

The prosthetic heart valve has a radially uncompressed condition and may include a plurality of hooks which are attached to the inner frame and which overlie the braided wire mesh when the valve is in the radially uncompressed condition. In such an embodiment the valve is orientated within the delivery capsule in the radially compressed condition such that the hooks are positioned radially inwardly of the braided wire mesh, and the braided wire mesh overlies the hooks.

The delivery device may be a catheter. Alternatively, the delivery device may be a capsule which is configured to retain the valve in radially compressed condition. The capsule may form an integral part of a catheter. Alternatively, the capsule may be mountable or otherwise connectable to a catheter

According to an example an example there is provided a method of unsheathing a prosthetic heart valve, the method comprising the steps of:
providing a delivery device having a prosthetic heart valve in a radially compressed condition located within the delivery device, wherein the prosthetic heart valve includes:
an inner frame having a tubular shape; and
a braided wire mesh arranged outside of the inner frame and defining a circumferential flange around the inner frame, the circumferential flange having an outer circumferential periphery; and
wherein the delivery device includes:
   a lumen defined by a wall of the delivery device, and a distal aperture;
   wherein the inner frame and braided wire mesh are maintained in the radially compressed condition by the wall; wherein
   the circumferential flange is held in an inverted state; and
   the part of the radially compressed prosthetic heart valve that is most proximal to the distal aperture of the delivery device is the portion of the braided wire mesh that defines the outer circumferential periphery of the flange;
   the method comprising the further steps of:
      longitudinally advancing the radially compressed prosthetic heart valve in the direction of the distal aperture of the delivery device such that the outer circumferential periphery of the inverted circumferential flange expands outwardly from the distal aperture; and
      continuing longitudinal advancement of the prosthetic heart valve to revert the circumferential flange to a non-inverted state and to fully unsheath the prosthetic heart valve from the delivery device.

Such a method of unsheathing the valve may be performed outside of the body of a patient for the purposes of familiarisation and training of medical practitioners.

The prosthetic heart valve may have a radially uncompressed condition and include a plurality of hooks which are attached to the inner frame and which overlie the braided wire mesh when the prosthetic heart valve is in the radially uncompressed condition; the prosthetic heart valve being orientated within the catheter or delivery capsule in the radially compressed condition such that the hooks are positioned radially inwardly of the braided wire mesh, and the braided wire mesh overlies the hooks;
wherein the step of continuing the longitudinal advancement of the prosthetic heart valve to revert the circumferential flange to the non-inverted state moves the hooks from the position where the hooks are radially inwardly of the braided wire mesh to the position where the hooks overlie the braided wire mesh.

The delivery device may be a catheter. Alternatively, the delivery device may be a capsule which is configured to retain the valve in radially compressed condition. The capsule may form an integral part of a catheter. Alternatively, the capsule may be mountable or otherwise connectable to a catheter

An embodiment of the present invention will now be described with reference to the accompanying figures in which;
Figure 1 depicts a cross section of a TMVR device in a radially uncompressed condition;
Figure 2 depicts a view from the upstream side to the downstream side of the device of figure 1 in a radially uncompressed condition;
Figure 3 depicts tapered parts of a hook of the device;
Figures 4, 5 and 6 depicts the device in a radially compressed condition in a capsule of a catheter;
Figure 7 depicts the device in a target condition positioned in a valve;
Figures 8 to 11 illustrate the loading of the device in a capsule of a catheter in accordance with an aspect of the invention;
Figure 12 depicts the device in a radially compressed condition in the capsule of a catheter, and in accordance with an aspect of the invention; and
Figures 13 to 17 depict the unsheathing of the device from the capsule of a catheter, and in accordance with an aspect of the present invention.

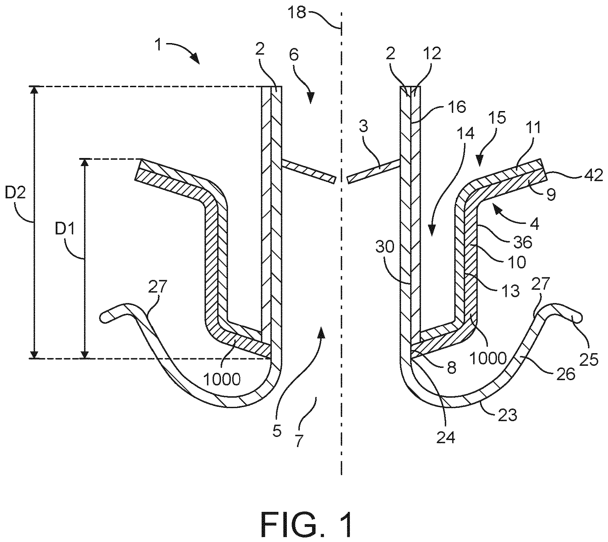

Referring to Fig. 1, a prosthetic heart valve 1 comprises an inner frame 2, two leaflets 3 and a braided wire mesh 1000. The heart valve 1 itself shown is described in greater detail in co-pending International patent application no PCT/EP2019/000309.

Fig. 1 depicts the prosthetic heart valve 1 in radially uncompressed condition. In the example shown, the prosthetic heart valve 1 is a prosthetic mitral valve suitable for transcatheter mitral valve replacement (TMVR). Moreover, when used according to the present invention, it is suitable for transseptal delivery. Description of the present invention with reference to TMVR is done by way of example only and is not intended to be limiting upon the scope of the claimed invention. As will be described in greater detail below, the present invention may be used in connection with other valve types and procedures.

The inner frame 2 is made by laser cutting mazes or apertures in a metal tube. The metal is a pseudoelastic metal, in this case nitinol, a nickel titanium metal alloy. Alternatively, the metal may be an alloy of Nickel and Cobalt.

The inner frame 2 has a tubular shape comprising a lumen 5 that extends from an upstream side 6 to a downstream side 7 of the prosthetic heart valve 1.

Upstream 6 side and downstream side 7 are used here even if the prosthetic heart valve is not implanted or even near a fluid as the prosthetic heart valve 1 is arranged to regulate fluid flow between the upstream 6 side and downstream side 7.

The radially uncompressed condition corresponds to a condition in which the prosthetic heart valve is not constrained radially and placed on a horizontal surface with its downstream side 7 or its upstream side 6 facing the horizontal surface.

The braided wire mesh 1000 is arranged outside the inner frame 2. The braided wire mesh forms a skeleton of a flange 4. The flange 4 comprises a single layer of a braided wire mesh 1000 that is coupled to the inner frame 2 at a coupling portion 8 of the flange. The braided wire mesh (and since that forms the skeleton of the flange 4 therefore the flange 4 also) further comprises a flared portion 9 and a body portion 10. The body portion 10 forms the connection between the flared portion 9 and the coupling portion 8. In the radially uncompressed condition, the flared portion 9 is closest of the 3 portions (i.e. the coupling portion 8, the body portion 10 and the flared portion 9) to the upstream side 6 and the coupling portion 8 is closest of the 3 portions to the downstream side 7.

The flange 4 further comprises a layer 11 of elastic material that is attached to the braided wire mesh 1000 by stitches (stitches not shown). Alternatively, the layer 11 of elastic material may be attached to the braided wire mesh 1000 by alternative means, for example by adhesive.

The elastic material is a polyurethane fabric. The braided wire mesh 1000 comprises a braided wire mesh surface 13 that at the body portion 10 faces the inner frame 2. The layer 11 of elastic material is attached to the braided wire mesh surface 13.

The prosthetic heart valve 1 forms a cavity 14 that is surrounded by the braided wire mesh 1000 (and the flange 4) and the inner frame 2. The inner frame 2 comprises an outer frame surface 16 that faces the cavity 14. The cavity 14 is not completely surrounded and has an opening 15 on the upstream side 6.

The lumen 5 is enclosed by a lumen wall. The lumen wall comprises the inner frame 2 and a further layer 12 of elastic material. The elastic material is a polyurethane fabric. The further layer 12 of elastic material is attached to the outer surface 16 by stitches (stitches not shown). Alternatively, the further layer 12 of elastic material may be attached to the outer surface 16 by alternative means, for example by adhesive.

The layer 11 of elastic material and the further layer 12 of elastic material are made from extensible fabric that has a low permeability for blood. The layer 11 of elastic material therefore forms a liner with low permeability for blood and the further layer 12 of elastic material therefore forms a further liner with low permeability for blood.

The permeability is chosen such that the material is not permeable below a blood pressure of at least 90 mm Hg which pressure corresponds to the pressure required to open the aortic valve.

At the coupling portion 8 the layer 11 of elastic material and the further layer 12 of elastic material are connected to each other by stitches to form a blood tight seam. The blood tight seam functions as a seal. The leaflets 3 are arranged in the lumen 5 and is arranged to regulate the fluid flow. The leaflets 3 preferably are of bovine pericardial tissue.

As the inner frame 2 and the flange 4 are lined with the layer 11 of elastic material and the further layer 12 of elastic material, fluids can only pass through the prosthetic heart valve 1 through the lumen and thus the leaflet assembly 3.

The inner frame 2 is made by laser cutting mazes in a metal tube such that the shape of the inner frame 2 in the radially uncompressed condition is constant along the axial direction. There may however be some effects, such as flaring or tapering at the ends after incorporation into the prosthetic valve 1.

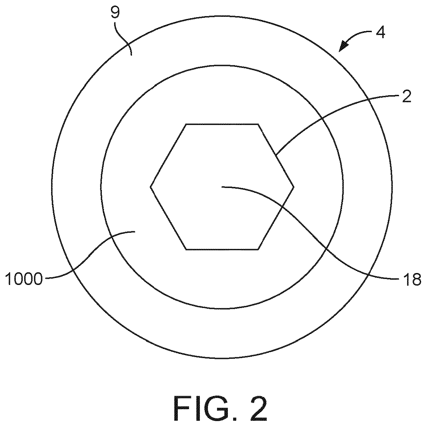

The inner frame 2 and the flange 4 each have a rotationally symmetric circumference around an axis 18 extending from the upstream side 6 to the downstream side 7. This is shown in figure 2. Having a rotationally symmetric circumference around the same axis, means that the inner frame 2 and the flange 4 are concentric.

The braided wire mesh 1000 is formed by a single continuous wire 17 which in the example shown is made from a pseudoelastic metal, in this case nitinol.

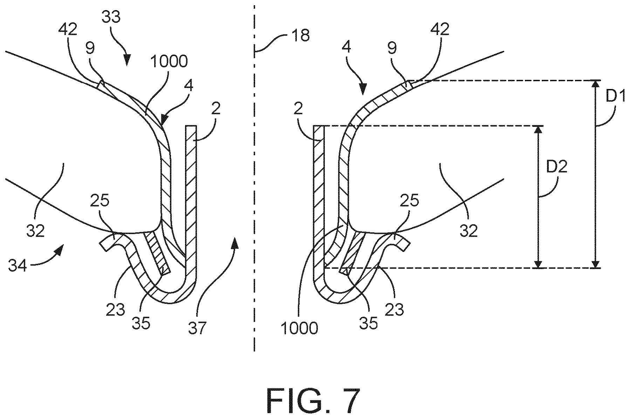

The prosthetic heart valve 1 further comprises a plurality of hooks 23 attached to the inner frame 2 on the downstream side 7. This is shown in figure 1. The hooks 23 point away from the lumen 5 and towards the upstream side 6. The hooks 23 are arranged to capture native leaflets 35 when unsheathed in a mitral valve annulus of a human heart. This is shown in figure 7. The hooks 23 are distributed at rotationally symmetric positions around the axis 18.

The hooks 23 each comprise an attachment end 24 where they are attached to the inner frame 2. The hooks 23 are formed together with the inner frame 2, however, they may be produced separately and attached to the inner frame 2 later. The hooks 23 further each comprise a top 25 at side of the hooks opposite to the attachment end 24.

The hooks 23 further each comprise a hook body which has an elongated shape. The hooks 23 comprise a surface 27 that at the hook body partially faces the flange 4. The hooks 23 are curved and the surface of the top 25 partially faces the upstream side 6 and partially faces radially outward.

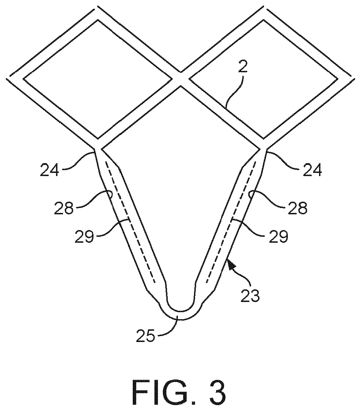

The hooks 23 each comprise two legs 28. This is shown in figure 3 which depicts a frontal view in the direction of the axis 18 where the hook 23 has been depicted as if it was made flat for the purpose of explanation, i.e. in the drawing the hook does not point away from the lumen 5 but the legs are parallel to the lumen 5.

The legs have a centre line 29 that extends between the attachment end 24 and the top 25. The dimension of the hook legs 28 perpendicular to the centre line 29 tapers towards the top 25.

Similarly, the dimension of the hook legs 28 perpendicular to the centre line 29 tapers at the attachment end 24 by becoming smaller towards the attachment end 24.

As shown in figure 1, the flange has a first dimension (D1) along the axis 18. The inner frame 2 has a second dimension (D2) along the axis 18. The second dimension (D2) is larger than the first dimension (D1) in this radially uncompressed condition.

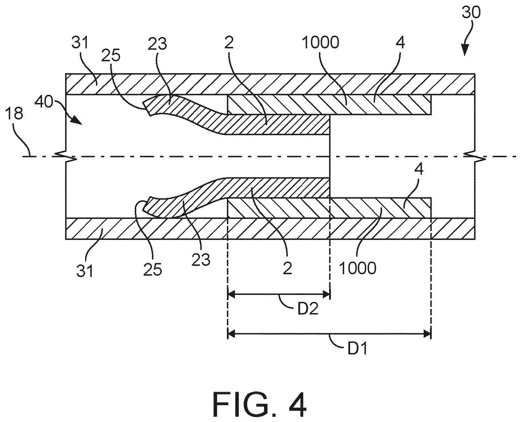

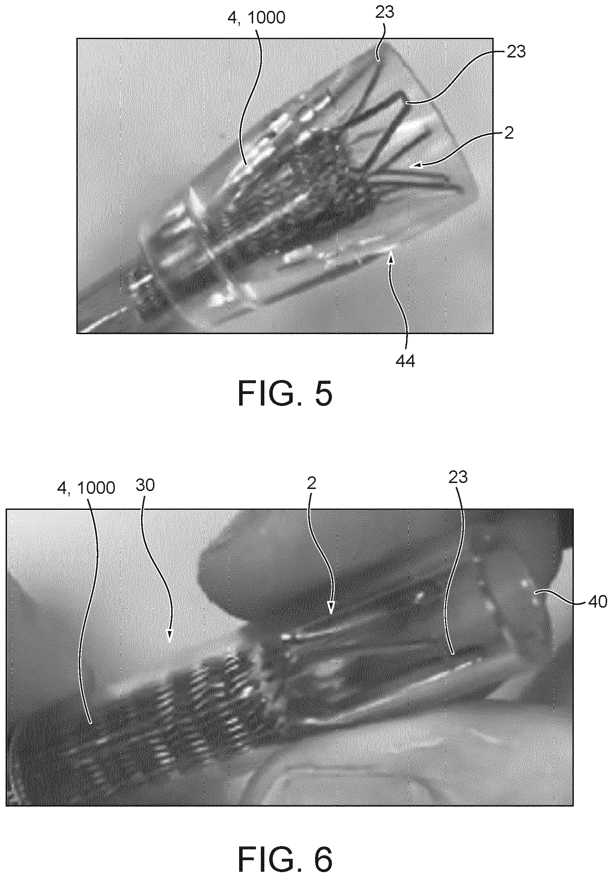

The valve 1 will now be described in a collapsed condition. This will be done with reference to figures 4 to 7. In these figures the prosthetic heart valve 1 is depicted in a delivery device such as a catheter 30. In figure 4 the layer 11 of elastic material and the further layer 12 of elastic material are not shown for clarity reasons.

The catheter 30 has a cylindrical catheter wall 31 defining a lumen. The collapsed condition is not a stable condition and can be maintained only as long as the prosthetic heart valve 1 stays inserted in the catheter 30 and is exposed to radial pressure by the catheter wall 31.

The inner frame 2 is radially collapsed over its entire length.

The braided wire mesh 1000 and thereby the flange 4 is also radially collapsed over the entire length in the axial direction of the braided wire mesh 1000. From the collapsed condition the prosthetic heart valve 1 can return to the radially uncompressed condition and the target condition without any permanent deformations because the inner frame 2 and the braided wire mesh 1000 are made of nitinol which has pseudoelastic properties. As the flange 4 comprises a layer 11 of elastic material to restrict the flow of blood, and the valve lumen wall comprises a further layer 12 elastic material, these layers are arranged to return to the radially uncompressed and target condition elastically as well.

In this case the hooks 23 are folded back and extend both away from the inner frame 2 and the flange 4.

In this collapsed state, the first dimension (D1) is larger than the second dimension (D2). This is the result of the braided wire mesh 1000 collapsing inwardly.

The valve 1 will now be described in a target condition. This will be done with reference to figure 7. In this figure the prosthetic heart valve 1 is depicted as unsheathed in the annulus 37 of a mitral valve in a human heart. The annulus 37 is an opening in the heart wall 32 separating the left atrium 33 and the left ventricle 34 of the human heart. In figure 7 the layer 11 of elastic material and the further layer 12 of elastic material are not shown for clarity reasons.

The target condition is a condition wherein only a part of the length of the body portion 10 of the flange 4 along the axis 18 is radially compressed.

A condition in which the prosthetic heart valve 1 is unsheathed and in use to regulate fluid flow, for instance as a mitral valve in a human heart corresponds to the target condition.

The heart wall 32 exerts radial pressure to the braided wire mesh 1000 of the flange 4 at the annulus 37. This pressure is not symmetrically applied and the shape of the heart wall 32 in the atrium is not constant around the axis 18. Therefore, the flared portion 9 reaches into the left atrium 33 differently around the axis 18.

Between the hooks 23 and the braided wire mesh 1000 two native leaflets 35 are captured. Even though the exerted radial pressure is asymmetric, the resulting radial compression of the braided wire mesh 1000 and thereby of the flange 4 causes the body portion 10 of the braided wire mesh 1000 to elongate in axial direction such that the first dimension (D1) increases.

The first dimension (D1) is larger than the second dimension (D2) in this target condition. In addition, the tops 25 of the hooks 23 are positioned against the heart wall 32. As the prosthetic heart valve 1 in radially uncompressed condition has the flared portion 9 closer to the hooks 23, the flared portion 9 exerts a force that pushes the top 25 of the hooks against the heart wall 32 which helps anchor the prosthetic heart valve 1.

It will be appreciated that the unsheathing of the valve 1 from the collapsed condition within the catheter 30 to the target condition in the mitral valve annulus 37 requires the catheter 30 to be positioned at least partially through the annulus 37 and into the ventricle 24. As noted above, this may not be possible for certain patients due to insufficient ventricular volume.

The conventional manner in which the valve 1 is unsheathed from the catheter 30, and described above with reference to figure 7, requires unsheathing of the hooks 23 prior to unsheathing of the braided wire mesh 1000. It will thus be understood that unsheathing of the valve 1 occurs initially within the ventricle 34 by the hooks 23, and subsequently in the atrium 33 by the braided wire mesh 1000. In accordance with the present invention however, these unsheathing steps are reversed such that initial unsheathing of the braided wire mesh 1000 occurs in the atrium 33 before subsequent unsheathing of the hooks 23 through the annulus 37 to the ventricle 34.

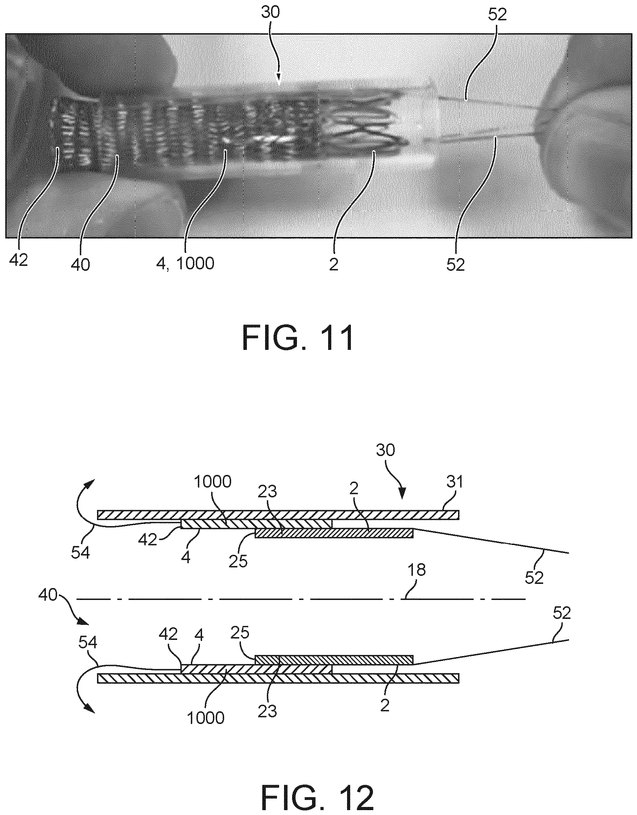

Unsheathing of the valve 1 in the manner described above is effected by altering the manner in which the valve 1 is loaded within the catheter 30 or, as will be described below, an alternative delivery device such as a capsule specifically configured to retain the valve 1 in a collapsed condition. More specifically, the valve 1 is loaded within the catheter 30 in a reversed and partially inverted orientation compared to the manner described above. Reference is made to figure 12 where features common to those described with reference to figure 4 are identified with like reference numerals. The catheter 30 includes a distal aperture 40 through which the valve 1 is inserted prior to introduction of the catheter 30 into a patient. The valve 1 is delivered through the distal aperture 40 of the catheter 30 when the valve 1 is unsheathed in the annulus of a mitral valve of a human heart.

As before, the catheter wall 31 exerts radial pressure on the valve 1 in order maintain both the inner frame 2 and braided wire mesh 1000 and thereby the flange 4 in the radially compressed condition. It will however be appreciated that the orientation of the valve 1 shown in figure 12 differs from that shown in figure 4. In figure 4, the valve 1 is positioned within the catheter 30 such that the tops 25 of the hooks 23 are proximal to the distal aperture 40 of the catheter 30. The hooks 23 extend both away from the inner frame 2 and flange 4 in the direction of the distal aperture 40 of the catheter.

Referring now to figure 12, the valve 1 is positioned within the catheter 30 with the circumferential flange inverted such that the braided wire mesh 1000 and flange 4 overlie the hooks 23. The hooks 23 are thus held radially inwardly of the flange 4. The portion of the valve 1 that is proximal to the distal aperture 40 of the catheter 30 is the portion of the flange 4 that defines the outer circumferential periphery 42 of the flange 4.

It will be appreciated that the orientation of the radially compressed valve 1 within the catheter 30 prevents close interaction of the leaflets 3 with the frame 2. The possibility of the leaflets 2 this being damaged or otherwise detrimentally affected by frame 2 is reduced.

The circumferential flange 4 is considered inverted as its orientation relative to inner frame 2 when the prosthetic heart valve 1 is in the radially compressed condition is opposite to that exhibited when the prosthetic heart valve 1 is in the radially uncompressed condition. More specifically, a circumferential surface of the flange 4 which faces inwardly and towards the inner frame 2 when the prosthetic heart valve 1 is in the radially uncompressed condition is repositioned so as to face radially outwardly and away from the inner frame 2 when the prosthetic heart valve 1 is in the radially compressed condition. Similarly, a circumferential surface of the flange 4 which faces outwardly and away from the inner frame 2 when the prosthetic heart valve 1 is in the radially uncompressed condition is repositioned so as to face radially inwardly and towards the inner frame 2 when the prosthetic heart valve is in the radially compressed condition.

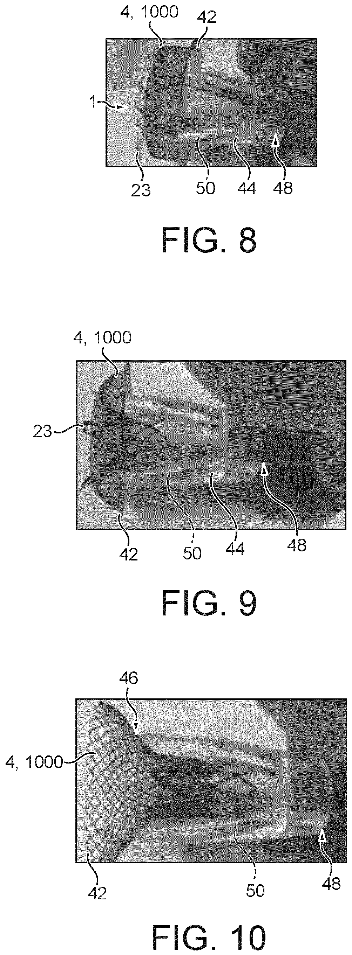

The manner in which the valve 1 is loaded into the catheter 30 is shown in figures 8 to 11. Figure 8 shows the valve 1 in the radially uncompressed condition positioned over a tapered conduit 44. The tapered conduit 44 is utilised to transition the valve 1 from the radially uncompressed condition to the collapsed condition within the catheter 30. The tapered conduit 44 has a first substantially circular opening or aperture 46, a second substantially circular opening or aperture 48 and a tapered lumen 50 extending there between. The first opening 46 has a greater diameter than the second opening 48. The diameter of the first opening 46 is greater than the uncompressed diameter of the inner frame 2 but less than the diameter of the flange 4. The diameter of the second opening 48 is approximately the same as the distal aperture 40 of the catheter 30. It will be understood that the taper of the lumen 50 between the openings 46,48 causes compression of the valve 1 as the valve 1 is drawn through the conduit from the first opening 46 to the second opening 48.

Referring again to figure 8, the valve 1 is positioned over the first opening of tapered conduit 44 such that the inner frame 2 is received through the first opening 46 and into the lumen 50, and the braided wire mesh 1000 and flange 4 extend around first opening 46. The tapered conduit 44 is provided adjacent the catheter such that the second opening of the tapered conduit 44 faces the distal opening 40 of the catheter 30. Removably attached to the inner frame 2, and extending through the tapered conduit 44, are traction wires 52 which are used to draw the valve 1 through the conduit 44. It will be understood that other methods may be employed to draw the valve 1 through the conduit 44. These may include, but not be limited to, manual manipulation, the use of a plunger tool, or the use of an iris crimper. The hooks 23 of the valve 1 can be seen overlying the exterior of the wire mesh 1000.

Figure 9 shows the position of the valve 1 after initial longitudinal movement of the inner frame 2 into the lumen 50 of the tapered conduit 44. The hooks 23 can still be seen to the exterior of the tapered conduit 44 and overlying the exterior of the wire mesh 1000.

Figure 10 shows the position of the valve 1 after further longitudinal movement of the inner frame 2 into lumen 50 of the tapered conduit 44. Radial compression of the inner frame 2 by the taper of the lumen 50 has commenced and the wire mesh 1000 has begun to be drawn into the lumen 50. It will be noted that the force applied to the wire mesh 1000 and flange 4 by the first opening 46 has caused inversion of the flange 4 such that the wire mesh 1000 now overlies the hooks 23 as opposed to the hooks 23 overlying the wire mesh 1000. It will be understood that continued longitudinal movement of the valve 1 through tapered lumen 50 causes radial compression of the inner frame 2 and wire mesh 1000 to a diameter equal to that of the second aperture 48 of the tapered conduit 44.

As noted above, the tapered conduit 44 is positioned adjacent the catheter 30, and the collapsed valve 1 can be thus be loaded into the catheter 30 through the distal aperture thereof by continued longitudinal movement. This is shown in figure 11.

The valve 1 may be loaded into the catheter 30 shortly prior to the scheduled surgical procedure to implant the valve 1 into a patient. As such, the loading of the valve 1 into the catheter 30 may be undertaken by a surgeon or a surgical practitioner during preparation for the implantation procedure.

In an alternative embodiment, the valve 1 may be supplied to a surgeon or surgical practitioner pre-loaded into a delivery capsule, which itself is then positioned in the catheter 30. In such an embodiment the delivery capsule may be provided with removable endcaps and contain a sterile liquid medium which surrounds the valve 1. The delivery capsule may further be provided with flushing ports to enable the sterile liquid medium to be removed from the delivery capsule. The manner in which the valve 1 is loaded into a delivery capsule is the same as that described above for the loading of a valve 1 into a catheter 30. The delivery capsule may be formed integrally with a catheter 30. Alternatively, the delivery capsule may be configured so as to be mountable or otherwise attachable to a catheter 30.

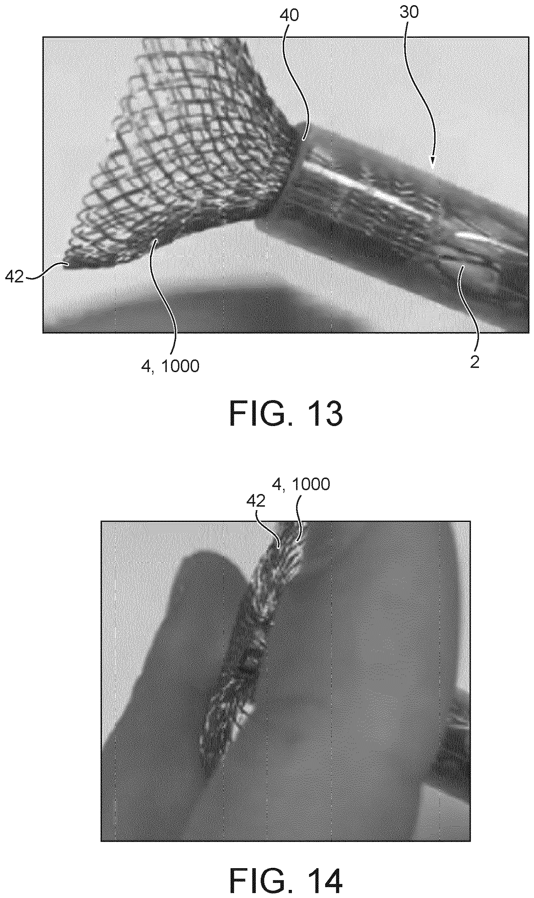

Figures 13 to 17 show the manner in which the valve 1 is unsheathed from the catheter 30. Unsheathing of the valve 1 is achieved by longitudinally advancing the collapsed valve 1 through the lumen of the catheter 30 and out of the distal aperture 40 of the catheter 30.

Figure 13 shows the initial unsheathing of the wire mesh 1000 through the distal aperture 40 of the catheter 30. Free from the constraints of the catheter wall 31, the mesh 1000 is able to radially expand as shown. Such radial expansion is driven by the inherent resilience of the mesh 1000 and/or shape memory attributes of the materials utilised for the mesh 1000 and inner frame 2. It will be noted however that the flange 4 is still in the inverted state, and that the outer circumferential periphery 42 of the flange 4 is projects distally of the distal aperture 40 of the catheter 30.

Delivery of the valve 1 requires the circumferential periphery 42 of the flange 4 to engage an annulus 37 separating the left atrium 33 and the left ventricle 34. This engagement is illustrated in figure 14 whereby spaced apart fingers simulate the annulus 37. It will be understood that the distal projection of the inverted flange 4 from the distal aperture 40 of the catheter 30 means that the catheter 30 can be located fully within the atrium 33, and thus does not need to be positioned through the annulus 37 and into the ventricle 34 in order to effect unsheathing of the valve 1. Supra-annular unsheathing of the valve 1 may thus be effected.

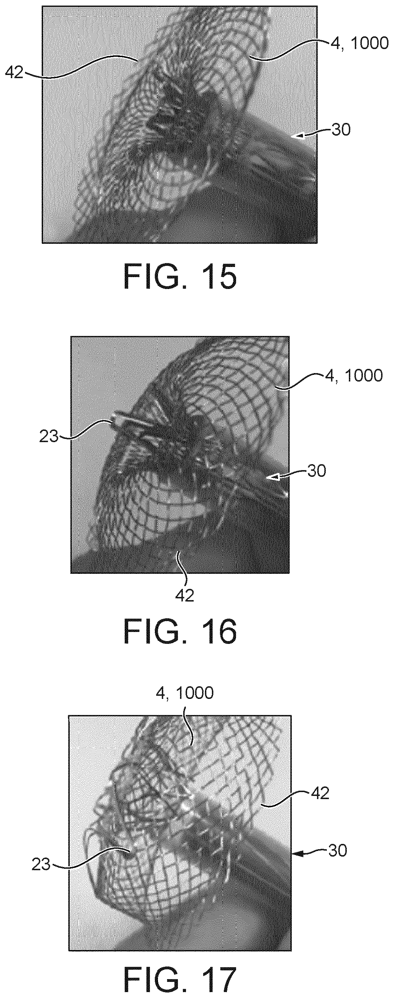

Figures 15 to 17 show the continued unsheathing of the valve 1 as the result of continued longitudinal advancement of the collapsed valve 1 through the lumen of the catheter 30 and out of the distal aperture 40 of the catheter 30. This includes the reversion of the flange 4 to its original non-inverted state (also indicated by arrows 54 on figure 12), and the re-positioning of the hooks 23 to the exterior of the braided wire mesh 1000.

The manner in which the valve 1 may be unsheathed from a delivery capsule corresponds to that described above with reference to the unsheathing of the valve 1 from a catheter 30.

As noted above, the above description of transcatheter mitral valve replacement is provided by way of example only and is not intended to be limiting upon the scope of the claimed invention. The manner in which the valve 1 is loaded into, and unsheathed from, the catheter or delivery capsule may equally be utilised in connection with other prosthetic heart valves including prosthetic aortic and prosthetic tricuspid valves.

## Claims

1. A method of loading a prosthetic heart valve (1) into a delivery device, the prosthetic heart valve (1) including:
an inner frame (2) having a tubular shape; and
a braided wire mesh (1000) arranged outside of the inner frame (2) and defining a circumferential flange (4) around the inner frame (2);
the method comprising the steps of:
providing a delivery device having a lumen defined by a wall and a distal aperture (40) at a distal end thereof, the lumen having a diameter that is less than that of the inner frame (2) and braided wire mesh (1000);
providing the prosthetic heart valve (1) in a radially uncompressed condition;
positioning the prosthetic heart valve (1) such that the inner frame (2) is received through the distal aperture (40) and into the lumen;
moving the prosthetic heart valve (1) fully into the lumen such that the inner frame (2) and braided wire mesh (1000) are radially compressed; wherein the step of moving the braided wire mesh (1000) into the lumen of the delivery device includes inverting the circumferential flange (4).

2. A method as claimed in claim 1 wherein the loading of the prosthetic heart valve (1) into the delivery device utilises a tapered conduit (44) having a first aperture (46), a second aperture (48) and a tapered lumen (50) extending between the first aperture (46) and the second aperture (48), wherein the diameter of the first aperture (46)is greater than that of the second aperture (48), and wherein further the diameter of the first aperture (46) is greater than the diameter of the inner frame (2) and less than the diameter of the circumferential flange (4), the method comprising the steps of:
positioning the prosthetic heart valve (1) relative to the first aperture (46) of the tapered conduit (44) such that inner frame (2) is received through the first aperture (46) and within the tapered lumen (50), and the circumferential flange (4) surrounds the first aperture (46) to the exterior of the tapered conduit (44);
aligning the second aperture (48) of the tapered conduit (44) with the distal aperture (40) of the delivery device;
moving the prosthetic heart valve (1) fully into the tapered lumen (50) such that the inner frame (2) and braided wire mesh (1000) are radially compressed by the tapered lumen (50) of the tapered conduit (44) before the compressed prosthetic heart valve (1) passes into the lumen of the delivery device through the distal aperture (40) of the delivery device.

3. A method as claimed as claimed in claim 2 wherein the step of inverting the circumferential flange (4) includes the step of moving the circumferential flange (4) into engagement with the portion of the tapered conduit (44) defining the first opening (46) thereof.

4. A method as claimed in any preceding claim wherein the prosthetic heart valve (1) includes a plurality of hooks (23) which are attached to the inner frame (2) and which overlie the braided wire mesh (1000) when the prosthetic heart valve (1) is in the radially uncompressed condition; wherein the step of inverting the circumferential flange (4) results in braided wire mesh (1000) moving to a position where it overlies the hooks (23).

5. A method as claimed in any preceding claim wherein the step of moving the prosthetic heart valve (1) into the lumen of the delivery device is achieved by the use of traction wires (52) which are releasably attachable to the inner frame (2) of the prosthetic heart valve (1).

6. A method as claimed in any preceding claim wherein the delivery device is a catheter (30).

7. A method as claimed in any of claims 1 to 5 wherein the delivery device is a capsule.

8. A method as claimed in claim 7 wherein the capsule forms an integral part of a catheter (30).

9. A method as claimed in claim 7 wherein the capsule is mountable or otherwise connectable to a catheter (30).

10. In combination, a delivery capsule and a prosthetic heart valve (1) in a radially compressed condition and located within the delivery device, wherein the prosthetic heart valve (1) includes:
an inner frame (2) having a tubular shape; and
a braided wire mesh (1000) arranged outside of the inner frame (2) and defining a circumferential flange (4) around the inner frame (2), the circumferential flange (4) having an outer circumferential periphery (42); and
wherein the delivery device includes:
a lumen defined by a wall of the delivery device, and a distal aperture (40);
wherein the inner frame (2) and braided wire mesh (1000) are maintained in the radially compressed condition by the wall;
the circumferential flange (4) is held in an inverted state; and
the part of the radially compressed prosthetic heart valve (1) that is most proximal to the distal aperture (40) of the delivery device is the portion of the braided wire mesh (1000) that defines the outer circumferential periphery (42) of the circumferential flange (4).

11. A combination as claimed in claim 10, wherein the part of the radially compressed prosthetic heart valve (1) that is most distal to the distal aperture (40) of the delivery device is the inner frame (2) of the prosthetic heart valve (1).

12. A combination as claimed in claim 10 or claim 11 wherein the prosthetic heart valve (1) has a radially uncompressed condition and includes a plurality of hooks (23) which are attached to the inner frame (2) and which overlie the braided wire mesh (1000) when the prosthetic heart valve is in the radially uncompressed condition; the prosthetic heart valve (1) being orientated within the delivery device in the radially compressed condition such that the hooks (23) are positioned radially inwardly of the braided wire mesh (1000), and the braided wire mesh (1000) overlies the hooks (23).

13. A combination as claimed in any of claims 10 to 12 wherein the delivery device is a catheter (30).

14. A combination as claimed in any of claims 10 to 12 wherein the delivery device is a capsule.

15. A combination as claimed in claim 14 wherein the capsule forms an integral part of a catheter (30).

16. A combination as claimed in claim 14 wherein the capsule is mountable or otherwise connectable to a catheter (30).

## Patentansprüche

1. Verfahren zum Laden einer Herzklappenprothese (1) in eine Zuführvorrichtung, wobei die Herzklappenprothese (1) Folgendes aufweist:
einen inneren Rahmen (2) mit einer Röhrenform und
ein geflochtenes Drahtgeflecht (1000), das außerhalb des inneren Rahmens (2) angeordnet ist und einen Umfangsflansch (4) um den inneren Rahmen (2) definiert, wobei das Verfahren die folgenden Schritte umfasst:
Bereitstellen einer Zuführvorrichtung mit einem Lumen, das durch eine Wand und eine distale Öffnung (40) an einem distalen Ende davon definiert ist, wobei das Lumen einen Durchmesser aufweist, der kleiner als der des inneren Rahmens (2) und des geflochtenen Drahtgeflechts (1000) ist,
Bereitstellen der Herzklappenprothese (1) in einem radial unkomprimierten Zustand,
Positionieren der Herzklappenprothese (1) derart, dass der innere Rahmen (2) durch die distale Öffnung (40) und in das Lumen aufgenommen wird,
vollständiges Bewegen der Herzklappenprothese (1) in das Lumen, so dass der innere Rahmen (2) und das geflochtene Drahtgeflecht (1000) radial komprimiert werden, wobei der Schritt des Bewegens des geflochtenen Drahtgeflechts (1000) in das Lumen der Zuführvorrichtung das Umstülpen des Umfangsflanschs (4) umfasst.

2. Verfahren nach Anspruch 1, wobei beim Laden der Herzklappenprothese (1) in die Zuführvorrichtung ein sich verjüngender Kanal (44) mit einer ersten Öffnung (46), einer zweiten Öffnung (48) und einem sich verjüngenden Lumen (50), das sich zwischen der ersten Öffnung (46) und der zweiten Öffnung (48) erstreckt, zum Einsatz kommt, wobei der Durchmesser der ersten Öffnung (46) größer als der der zweiten Öffnung (48) ist und wobei ferner der Durchmesser der ersten Öffnung (46) größer als der Durchmesser des inneren Rahmens (2) und kleiner als der Durchmesser des Umfangsflanschs (4) ist, wobei das Verfahren die folgenden Schritte umfasst:
Positionieren der Herzklappenprothese (1) bezüglich der ersten Öffnung (46) des sich verjüngenden Kanals (44), so dass der innere Rahmen (2) durch die erste Öffnung (46) und in dem sich verjüngenden Lumen (50) aufgenommen wird und der Umfangsflansch (4) die erste Öffnung (46) zu der Außenseite des sich verjüngenden Kanals (44) umgibt,
Ausrichten der zweiten Öffnung (48) des sich verjüngenden Kanals (44) auf die distale Öffnung (40) der Zuführvorrichtung,
vollständiges Bewegen der Herzklappenprothese (1) in das sich verjüngende Lumen (50), so dass der innere Rahmen (2) und das geflochtene Drahtgeflecht (1000) durch das sich verjüngende Lumen (50) des sich verjüngenden Kanals (44) radial komprimiert werden, bevor die komprimierte Herzklappenprothese (1) durch die distale Öffnung (40) der Zuführvorrichtung in das Lumen der Zuführvorrichtung gelangt.

3. Verfahren nach Anspruch 2, wobei der Schritt des Umstülpens des Umfangsflanschs (4) den Schritt des Bewegens des Umfangsflanschs (4) in Eingriff mit dem Abschnitt des sich verjüngenden Kanals (44), der die erste Öffnung (46) davon definiert, aufweist.

4. Verfahren nach einem der vorhergehenden Ansprüche, wobei die Herzklappenprothese (1) eine Vielzahl von Haken (23) aufweist, die an dem inneren Rahmen (2) angebracht sind und die über dem geflochtenen Drahtgeflecht (1000) liegen, wenn sich die Herzklappenprothese (1) in dem radial unkomprimierten Zustand befindet, wobei der Schritt des Umstülpens des Umfangsflanschs (4) dazu führt, dass sich das geflochtene Drahtgeflecht (1000) in eine Position bewegt, in der es über dem Haken (23) liegt.

5. Verfahren nach einem der vorhergehenden Ansprüche, wobei der Schritt des Bewegens der Herzklappenprothese (1) in das Lumen der Zuführvorrichtung durch die Verwendung von Traktionsdrähten (52) erzielt wird, die lösbar an dem inneren Rahmen (2) der Herzklappenprothese (1) anbringbar sind.

6. Verfahren nach einem der vorhergehenden Ansprüche, wobei die Zuführvorrichtung ein Katheter (30) ist.

7. Verfahren nach einem der Ansprüche 1 bis 5, wobei die Zuführvorrichtung eine Kapsel ist.

8. Verfahren nach Anspruch 7, wobei die Kapsel einen integralen Teil eines Katheters (30) bildet.

9. Verfahren nach Anspruch 7, wobei die Kapsel an einen Katheter (30) montierbar oder anderweitig mit diesem verbindbar ist.

10. Zuführkapsel und Herzklappenprothese (1) in Kombination in einem radial komprimierten Zustand und in der Zuführvorrichtung angeordnet, wobei die Herzklappenprothese (1) Folgendes aufweist:
einen inneren Rahmen (2) mit einer Röhrenform und
ein geflochtenes Drahtgeflecht (1000), das außerhalb des inneren Rahmens (2) angeordnet ist und einen Umfangsflansch (4) um den inneren Rahmen (2) definiert, wobei der Umfangsflansch (4) eine äußere Umfangsperipherie (42) aufweist, und
wobei die Zuführvorrichtung Folgendes aufweist:
ein Lumen, das durch eine Wand der Zuführvorrichtung definiert ist, und eine distale Öffnung (40),
wobei der innere Rahmen (2) und das geflochtene Drahtgeflecht (1000) durch die Wand in dem radial komprimierten Zustand gehalten werden,
der Umfangsflansch (4) in einem umgestülpten Zustand gehalten wird und
der Teil der radial komprimierten Herzklappenprothese (1), der am proximalsten zu der distalen Öffnung (40) der Zuführvorrichtung liegt, der Teil des geflochtenen Drahtgeflechts (1000) ist, der die äußere Umfangsperipherie (42) des Umfangsflanschs (4) definiert.

11. Kombination nach Anspruch 10, wobei der Teil der radial komprimierten Herzklappenprothese (1), der am distalsten zu der distalen Öffnung (40) der Zuführvorrichtung liegt, der innere Rahmen (2) der Herzklappenprothese (1) ist.

12. Kombination nach Anspruch 10 oder Anspruch 11, wobei die Herzklappenprothese (1) einen radial unkomprimierten Zustand hat und eine Vielzahl von Haken (23) aufweist, die an dem inneren Rahmen (2) angebracht sind und die über dem geflochtenen Drahtgeflecht (1000) liegen, wenn sich die Herzklappenprothese in dem radial unkomprimierten Zustand befindet, wobei die Herzklappenprothese (1) in der Zuführvorrichtung in dem radial komprimierten Zustand ausgerichtet ist, so dass die Haken (23) radial einwärts von dem geflochtenen Drahtgeflecht (1000) positioniert sind und das geflochtene Drahtgeflecht (1000) über den Haken (23) liegt.

13. Kombination nach einem der Ansprüche 10 bis 12, wobei die Zuführvorrichtung ein Katheter (30) ist.

14. Kombination nach einem der Ansprüche 10 bis 12, wobei die Zuführvorrichtung eine Kapsel ist.

15. Kombination nach Anspruch 14, wobei die Kapsel einen integralen Teil eines Katheters (30) bildet.

16. Kombination nach Anspruch 14, wobei die Kapsel an einen Katheter (30) montierbar oder anderweitig damit verbindbar ist.

## Revendications

1. Procédé de chargement d'une valvule cardiaque prothétique (1) dans un dispositif d'administration, la valvule cardiaque prothétique (1) comprenant :
un cadre interne (2) ayant une forme tubulaire ; et
un treillis de fils tressés (1000) agencé à l'extérieur du cadre interne (2) et définissant une bride circonférentielle (4) autour du cadre interne (2) ;
le procédé comprenant les étapes de :
fourniture d'un dispositif d'administration ayant une lumière définie par une paroi et une ouverture distale (40) à une extrémité distale de celle-ci, la lumière ayant un diamètre qui est inférieur à celui du cadre interne (2) et du treillis de fils tressés (1000) ;
fourniture de la valvule cardiaque prothétique (1) dans un état radialement non comprimé ;
positionnement de la valvule cardiaque prothétique (1) de manière à ce que le cadre interne (2) soit reçu à travers l'ouverture distale (40) et dans la lumière ;
déplacement de la valvule cardiaque prothétique (1) entièrement dans la lumière de sorte que le cadre interne (2) et le treillis de fils tressés (1000) soient comprimés radialement ; l'étape de déplacement du treillis de fils tressés (1000) dans la lumière du dispositif d'administration comprenant l'inversion de la bride circonférentielle (4).

2. Procédé selon la revendication 1, le chargement de la valvule cardiaque prothétique (1) dans le dispositif d'administration utilisant un conduit conique (44) ayant une première ouverture (46), une deuxième ouverture (48) et une lumière conique (50) s'étendant entre la première ouverture (46) et la deuxième ouverture (48), le diamètre de la première ouverture (46) étant supérieur à celui de la seconde ouverture (48), et en outre le diamètre de la première ouverture (46) étant supérieur au diamètre du cadre interne (2) et inférieur au diamètre de la bride circonférentielle (4), le procédé comprenant les étapes de :
positionnement de la valvule cardiaque prothétique (1) par rapport à la première ouverture (46) du conduit conique (44) de sorte que le cadre interne (2) soit reçu à travers la première ouverture (46) et à l'intérieur de la lumière conique (50), et que la bride circonférentielle (4) entoure la première ouverture (46) vers l'extérieur du conduit conique (44) ;
alignement de la seconde ouverture (48) du conduit conique (44) avec l'ouverture distale (40) du dispositif d'administration ;
déplacement de la valvule cardiaque prothétique (1) complètement dans la lumière conique (50) de sorte que le cadre interne (2) et le treillis de fils tressés (1000) soient comprimés radialement par la lumière conique (50) du conduit conique (44) avant que la valvule cardiaque prothétique (1) comprimée ne passe dans la lumière du dispositif d'administration par l'ouverture distale (40) du dispositif d'administration.

3. Procédé selon la revendication 2, l'étape d'inversion de la bride circonférentielle (4) comprenant l'étape de déplacement de la bride circonférentielle (4) en prise avec la partie du conduit conique (44) définissant la première ouverture (46) de celui-ci.

4. Procédé selon l'une quelconque des revendications précédentes, la valvule cardiaque prothétique (1) comprenant une pluralité de crochets (23) qui sont fixés au cadre interne (2) et qui recouvrent le treillis de fils tressés (1000) lorsque la valvule cardiaque prothétique (1) est dans l'état radialement non comprimé ; l'étape d'inversion de la bride circonférentielle (4) entraînant le déplacement du treillis de fils tressés (1000) vers une position où il recouvre les crochets (23).

5. Procédé selon l'une quelconque des revendications précédentes, l'étape de déplacement de la valvule cardiaque prothétique (1) dans la lumière du dispositif d'administration étant réalisée par l'utilisation de fils de traction (52) qui sont attachés de manière libérable au cadre interne (2) de la valvule cardiaque prothétique (1).

6. Procédé selon l'une quelconque des revendications précédentes, le dispositif d'administration étant un cathéter (30).

7. Procédé selon l'une quelconque des revendications 1 à 5, le dispositif d'administration étant une capsule.

8. Procédé selon la revendication 7, la capsule faisant partie intégrante du cathéter (30).

9. Procédé selon la revendication 7, la capsule pouvant être montée ou autrement connectée à un cathéter (30).

10. En combinaison, capsule d'administration et valvule cardiaque prothétique (1) dans un état comprimé radialement et située à l'intérieur du dispositif d'administration, la valvule cardiaque prothétique (1) comprenant :
un cadre interne (2) ayant une forme tubulaire ; et
un treillis de fils tressés (1000) agencé à l'extérieur du cadre interne (2) et définissant une bride circonférentielle (4) autour du cadre interne (2), la bride circonférentielle (4) ayant une périphérie circonférentielle externe (42) ; et
le dispositif d'administration comprenant :
une lumière définie par une paroi du dispositif d'administration, et une ouverture distale (40) ;
le cadre interne (2) et le treillis de fils tressés (1000) étant maintenus en état de compression radiale par la paroi ;
la bride circonférentielle (4) étant maintenue à l'état inversé ; et
la partie de la valvule cardiaque prothétique (1) comprimée radialement qui est la plus proximale de l'ouverture distale (40) du dispositif d'administration étant la partie du treillis de fils tressés (1000) qui définit la périphérie circonférentielle externe (42) de la bride circonférentielle (4).

11. Combinaison selon la revendication 10, la partie de la valvule cardiaque prothétique (1) comprimée radialement qui est la plus distale par rapport à l'ouverture distale (40) du dispositif d'administration étant le cadre interne (2) de la valvule cardiaque prothétique (1).

12. Combinaison selon la revendication 10 ou la revendication 11, la valvule cardiaque prothétique (1) ayant un état radialement non comprimé et comprenant une pluralité de crochets (23) qui sont fixés au cadre interne (2) et qui recouvrent le treillis de fils tressés (1000) lorsque la valvule cardiaque prothétique est dans l'état radialement non comprimé ; la valvule cardiaque prothétique (1) étant orientée à l'intérieur du dispositif d'administration à l'état comprimé radialement de sorte que les crochets (23) sont positionnés radialement vers l'intérieur du treillis de fils tressés (1000), et que le treillis de fils tressés (1000) recouvre les crochets (23).

13. Combinaison selon l'une quelconque des revendications 10 à 12, le dispositif d'administration étant un cathéter (30).

14. Combinaison selon l'une quelconque des revendications 10 à 12, le dispositif d'administration étant une capsule.

15. Combinaison selon la revendication 14, la capsule faisant partie intégrante du cathéter (30).

16. Combinaison selon la revendication 14, la capsule pouvant être montée ou connectée d'une autre manière à un cathéter (30).
